**Europäisches Patentamt**

**European Patent Office**

**Office européen des brevets**

(19)

(11) Veröffentlichungsnummer: **0 111 147 B1**

(12) ## EUROPÄISCHE PATENTSCHRIFT

(45) Veröffentlichungstag der Patentschrift:
**14.05.86**

(21) Anmeldenummer: **83110894.9**

(22) Anmeldetag: **02.11.83**

(51) Int. Cl.⁴: **C 01 B 33/28,** B 01 J 29/28, C 07 C 1/20, C 07 C 11/20

(54) **Titan-, zirkon- und/oder hafniumhaltige Zeolithe und Verfahren zu ihrer Herstellung sowie ihre Verwendung.**

(30) Priorität: **05.11.82 DE 3240870**

(43) Veröffentlichungstag der Anmeldung:
**20.06.84 Patentblatt 84/25**

(45) Bekanntmachung des Hinweises auf die Patenterteilung:
**14.05.86 Patentblatt 86/20**

(84) Benannte Vertragsstaaten:
**BE DE FR GB IT NL**

(56) Entgegenhaltungen:
**DE - A - 2 749 024**
**DE - A - 3 136 684**
**DE - A - 3 136 686**
**DE - A - 3 141 285**
**DE - A - 3 217 322**
**DE - A - 3 217 323**
**US - A - 2 950 952**

(73) Patentinhaber: **HOECHST AKTIENGESELLSCHAFT,
Postfach 80 03 20, D-6230 Frankfurt am Main 80 (DE)**

(72) Erfinder: **Baltes, Herbert, Dr., Johannesallee 24,
D-6230 Frankfurt am Main 80 (DE)**
Erfinder: **Litterer, Heinz, Dr., Albert-Schweitzer-Allee 39,
D-6200 Wiesbaden (DE)**
Erfinder: **Leupold, Ernst Ingo, Dr., Am Zäunefeld 15,
D-6392 Neu-Anspach (DE)**
Erfinder: **Wunder, Friedrich, Dr., Jahnstrasse 46,
D-6093 Flörsheim am Main (DE)**
Erfinder: **Ebertz, Wolfgang, Dr., Nelkenweg 12,
D-5047 Wesseling (DE)**

## Beschreibung

Als Zeolithe bezeichnet man vor allem kristalline Aluminosilicate, bei denen durch eine dreidimensionale Verknüpfung von $SiO_4$- und $AlO_4$-Tetraedern regelmässige Strukturen mit Hohlräumen und Poren entstehen. Im hydratisierten Zustand sind diese Poren und Hohlräume mit Wasser gefüllt. Dieses lässt sich ohne Beeinflussung der Kristallstruktur entfernen oder durch andere Moleküle ersetzen. Die negativen Ladungen der $AlO_4$-Tetraeder werden durch Kationen kompensiert. Diese können, wenn gewünscht, gegen andere Kationen ausgetauscht werden. Die geschilderten Eigenschaften ermöglichen die Verwendung der Zeolithe als Ionenaustauscher, Adsorbentien und Katalysatoren (D.W. Breck: Zeolite Molecular Sieves, 1974).

Zeolithe des X-, Y, Mordenit-, Erionit- und Offretit-Typs beispielsweise besitzen als Katalysatoren für Umwandlungsreaktionen von Kohlenwasserstoffen wie Cracken, Hydrocracken oder Isomerisierungen beträchtliches technisches Interesse. Zeolithe vom Pentasil-Typ (z. B. Zeolith ZSM-5) gewinnen als Katalysatoren für die Umwandlung von Methanol zu Kohlenwasserstoffen steigende Bedeutung.

Aufgrund der zahlreichen Einsatzmöglichkeiten als Katalysatoren besteht grosses Interesse an neuen Zeolithen mit spezifischen katalytischen Eigenschaften. Beispielsweise erhält man sehr interessante Zeolithe, wenn man anstelle von Aluminium und/oder Silicium andere Elemente in das Zeolith-Gerüst einbaut. So wurden unter anderem Zeolithe der Pentasil-Reihe bekannt, die Bor (DE-A-2 830 787), Eisen (DE-A-2 831 611), Arsen (DE-B-2 830 830), Antimon (DE-A-2 830 787), Vanadium (DE-A-2 831 631), Chrom (DE-A-2 831 630) oder Gallium (BE-A-882 484) auf Tetraederplätzen enthalten.

Auch wurden Titanosilicate (US-A-3 329 481 und DE-A-3 047 798) und Zirkonosilicate (US-A-3 329 480) mit Zeolithstruktur bekannt.

Weiter wurden bereits beschrieben: borhaltige Zeolithe, gallium- und/oder indiumhaltige Zeolithe, titanhaltige Zeolithe sowie zirkon- und/oder hafniumhaltige Zeolithe (deutsche Offenlegungsschriften DE-A-3 134 316, 3 134 317, 3 136 686, 3 136 684, 3 141 283, 3 141 285, 3 217 324, 3 217 323).

Gegenstand der Erfindung sind titan-, zirkon- und/oder hafniumhaltige Zeolithe, die dadurch gekennzeichnet sind, dass sie

a) ausser Natrium und Kalium die Element Silicium, Aluminium sowie mindestens ein Element aus der Gruppe Titan, Zirkon und Hafnium in folgendem Verhältnis enthalten, ausgedrückt in Molverhältnissen von Oxiden:

$$(SiO_2 + MO_2) : (0{,}02 - 0{,}30)Al_2O_3,$$

wobei M gleich Titan, Zirkon und/oder Hafnium ist,
und

b) im Röntgenbeugungsdiagramm die in Tabelle 1 aufgeführten charakteristischen Signale aufweisen:

*Tabelle 1*

| Netzebenenabstände d (Å) | relative Intensität $I/I_o$ |
|---|---|
| 11,4 ± 0,3 | stark bis sehr stark |
| 9,2 ± 0,2 | schwach |
| 7,6 ± 0,2 | schwach bis mittel |
| 6,6 ± 0,1 | mittel bis stark |
| 5,7 ± 0,1 | schwach bis mittel |
| 5,35 ± 0,1 | schwach |
| 4,56 ± 0,1 | mittel bis stark |
| 4,32 ± 0,1 | stark |
| 4,16 ± 0,1 | schwach |
| 3,81 ± 0,1 | mittel bis stark |
| 3,75 ± 0,1 | stark bis sehr stark |
| 3,59 ± 0,1 | stark bis sehr stark |
| 3,30 ± 0,1 | mittel |
| 3,15 ± 0,1 | mittel |
| 2,86 ± 0,1 | stark bis sehr stark |
| 2,80 ± 0,1 | schwach bis mittel |

Hierbei bedeutet $I_o$ die Intensität des stärksten Signals, und für das Verhältnis von Silicium zu Titan, Zirkon und/oder Hafnium gilt

$$\frac{SiO_2}{SiO_2 + MO_2} = 0.4\text{-}0.99,$$

ausgedrückt in Molverhältnissen der Oxide. Für die Angabe der Intensität in Tabelle 1 gilt:

| relative Intensität | $100\ I/I_o$ |
|---|---|
| sehr stark | 80-100 |
| stark | 50- 80 |
| mittel | 20- 50 |
| schwach | 0- 20 |

Für das Verhältnis von Silicium zu Titan, Zirkon und/oder Hafnium in den erfindungsgemässen Zeolithen gilt vorzugsweise

$$\frac{SiO_2}{SiO_2 + MO_2} = 0{,}7\text{-}0{,}99,$$

ausgedrückt in Molverhältnissen der Oxide, wobei M gleich Titan, Zirkon und/oder Hafnium ist.

Die Gitterstruktur der erfindungsgemässen Zeolithe ähnelt denen der folgenden synthetischen Zeolithe:

— Zeolith T (US-A-2 950 952)
— Zeolith ZSM-34 (DE-A-2 749 024)
— Aluminosilicate-Zeolith gemäss der DE-A-3 217 322.

Von diesen drei genannten synthetischen Zeolithen unterscheiden sich die erfindungsgemässen Zeolithe durch ihre Zusammensetzung, insbesondere durch den Gehalt an mindestens einem Element aus der Gruppe Titan, Zirkon und Hafnium.

Von den synthetischen Zeolithen gemäss DE-A-2 749 024 und DE-A-3 217 322 unterscheiden sich die erfindungsgemässen Zeolithe ausserdem durch das Fehlen organischer Bestandteile („Schablonenverbindungen" oder „Template") wie Cholinverbindungen oder anderer Hydroxyalkylammoniumverbindungen.

Die titan- bzw. zirkon- und/oder hafniumhaltigen Zeolithe gemäss den deutschen Offenlegungsschriften DE-A-31 36 686 bzw. 31 36 684 besitzen ebenfalls eine den erfindungsgemässen Zeolithen ähnliche Struktur, unterscheiden sich vom letzteren jedoch durch ihren Gehalt an Cholinverbindungen. Diese Cholinverbindungen dienen während der Synthese in der Regel als Template (Schablonenverbindungen) und beeinflussen die Struktur. Nach der Synthese werden sie im allgemeinen durch Kalzinieren unter Zersetzung aus dem Kristallgitter entfernt. Die erfindungsgemässen titan-, zirkon- und/oder hafniumhaltigen Zeolithe zeichnen sich somit durch zwei wesentliche Vorteile gegenüber den Zeolithen gemäss der DE-A-31 36 686 und DE-A 31 36 684 aus:

— Während der Synthese kann auf den Zusatz von teuren Cholinverbindungen verzichtet werden
— Die Kalzinierung des Syntheseproduktes erübrigt sich.

Von den Titanosilicaten gemäss US-A-3 329 481 und DE-A-3 047 798, den Zirkonosilicaten gemäss US-A-3 329 480, den titanhaltigen Zeolithen gemäss der DE-A-31 41 283 sowie den zirkon- und/oder hafniumhaltigen Zeolithen gemäss der DE-A-31 41 285 unterscheiden sich die erfindungsgemässen titan-, zirkon- und/oder hafniumhaltigen Zeolithe durch die Struktur sowie durch das Fehlen einer organischen Ammoniumverbindung.

Die erfindungsgemässen Zeolithe lassen sich herstellen, indem man Titan-, Zirkon- und/oder Hafniumverbindungen mit Aluminium-, Silicium-, Natrium-, Kaliumverbindungen und Wasser mischt und diese Mischung in einem geschlossenen Gefäss erhitzt. Vorzugsweise setzt man diesem Gemisch vor dem Erhitzen Impfkristalle eines Zeoliths eines ähnlichen Strukturtyps zu.

Die Ausgangsverbindungen werden in folgendem Verhältnis eingesetzt, ausgedrückt in Molverhältnissen der Oxide:

$(SiO_2 + MO_2): (0,02 - 0,30)Al_2O_3: (0,05 - 0,70)Na_2O: (0,02 - 0,30)K_2O: (5 - 90)H_2O,$

vorzugsweise im Verhältnis

$(SiO_2 + MO_2): (0,02 - 0,18)Al_2O_3: (0,10 - 0,60)Na_2O: (0,04 - 0,20)K_2O: (5 - 40)H_2O,$

wobei M gleich Titan, Zirkon und/oder Hafnium ist. Für das Verhältnis Silicium zu Titan, Zirkon und/oder Hafnium im Gemisch der Ausgangsverbindungen gilt

$$\frac{SiO_2}{SiO_2 + MO_2} = 0,4\text{-}0,99,$$

vorzugsweise

$$\frac{SiO_2}{SiO_2 + MO_2} = 0,6\text{-}0,99,$$

ausgedrückt in Molverhältnissen der Oxide, wobei M gleich Titan, Zirkon und/oder Hafnium ist.

Als Titan-, Zirkon- und Hafniumverbindungen können beispielsweise eingesetzt werden: Titanhalogenide, Titansulfat, Titanoxidsulfat, Titanalkoholate, Natriumtitanat, Kaliumtitanat, Titandioxid, Zirkonhalogenide, Zirkonsulfat, Zirkonalkoholate, Zirkonnitrat, Zirkondioxid, Zirkonylhalogenide, Zirkonylsulfat, Natriumzirkonat, Kaliumzirkonat, Hafniumhalogenide, Hafniumdioxid, Hafniumoxychlorid. Aber auch andere Titan-, Zirkonund Hafniumverbindungen eignen sich für die Herstellung der erfindungsgemässen Zeolithe.

Als Silicium-, Aluminium-, Natrium- und Kaliumverbindungen können beispielsweise eingesetzt werden: Kieselsäuregel, Kaliumsilicat, Natriumsilicat, Natriumaluminat, Kaliumaluminat, Aluminiumhalogenide, Aluminiummetahydroxid, Kaliumhydroxid, Kaliumsulfat, Kaliumhalogenide, Natriumhydroxid, Natriumsulfat, Natriumhalogenide. Aber auch andere Silicium-, Aluminium-, Kalium- und Natriumverbindungen eignen sich für die Herstellung der erfindungsgemässen Zeolithe.

Falls Impfkristalle verwendet werden, setzt man sie im allgemeinen in einer Menge von 0,1 bis 20 Gew.-% zu, vorzugsweise in einer Menge von 0,5 bis 10 Gew.-%, bezogen auf die Gesamtmenge aller anderen Komponenten des Reaktionsgemisches. Als Impfkristalle eignen sich alle Zeolithe mit gleicher oder ähnlicher Gitterstruktur, beispielsweise synthetische Zeolithe gemäss den deutschen Offenlegungsschriften DE-A-32 17 322, 31 36 686 und 31 36 684 oder natürlicher Erionit.

Das Gemisch der jeweils gewählten Verbindungen mit Wasser wird im allgemeinen 48 bis 2000 Stunden, vorzugsweise 48 bis 1 000 Stunden lang, auf eine Temperatur zwischen 80 und 160° C, vorzugsweise zwischen 90 und 150° C, in einem geschlossenen Gefäss erhitzt.

Die gebildeten Zeolithe werden in üblicher Weise, z. B. durch Filtration, isoliert, gewaschen und getrocknet. Sie können nach bekannten Methoden in die katalytisch aktiven Formen überführt werden, z. B. durch Ionenaustausch (D.W. Breck, Zeolite Molecular Sieves, 1974).

Die erfindungsgemässen Zeolithe zeichnen sich nach ihrer Überführung in die katalytisch aktiven Formen insbesondere aus durch eine hohe Selektivität und durch eine geringe Koksabscheidung bei der Umwandlung von Methanol in niedere Olefine. Diese Reaktion führt man beispielsweise bei Temperaturen von 350-430° C und einem Wasseranteil im Methanol von 0 bis 80 Gew.-% oder mit Rohmethanol durch:

Die Erfindung soll durch die folgenden Beispiele erläutert werden, wobei die Beispiele aber in keiner Weise einschränkend sein sollen. Alle angegebenen Röntgenbeugungsdaten wurden mit einem computergesteuerten Pulverdiffraktometer D-500 der Firma Siemens aufgenommen. Es wurde Kupfer-K-alpha-Strahlung verwandt.

*Beispiel 1*

18,4 g Natriumaluminat (54 Gew.-% $Al_2O_3$, 41 Gew.-% $Na_2O$), 23,5 g Natriumhydroxid und 8,6 g Kaliumhydroxid werden in 130 g Wasser gelöst. In diese Lösung werden unter intensivem Rühren nacheinander zuerst 203 g 40 Gew.-%iges kolloidales Kieselgel, anschliessend 16,4 g Titanethanolat $Ti(OC_2H_5)_4$ und zuletzt 4 g Impfkristalle (natürlicher Erionit) eingebracht. Die entstandene Mischung wird homogenisiert und 192 Stunden im geschlossenen Gefäss auf 155° C erhitzt. Das entstandene Produkt wird abfiltriert, mit Wasser gewaschen und bei 120° C getrocknet.

Das Produkt enthält Silicium, Aluminium und Titan in folgenden Anteilen, ausgedrückt in Molverhältnissen von Oxiden:

$$SiO_2 : 0,068\ Al_2O_3 : 0,084\ TiO_2.$$

Das Ergebnis der Röntgenbeugungsanalyse ist in der Tabelle 2 wiedergegeben.

*Tabelle 2*

| Netzebenenabstände d (Å) | relative Intensität 100 $I/I_o$ |
|---|---|
| 11,48 | 100 |
| 9,20 | 3 |
| 7,58 | 16 |
| 6,64 | 44 |
| 6,35 | 14 |
| 5,75 | 21 |
| 5,35 | 3 |
| 4,58 | 43 |
| 4,34 | 51 |
| 4,16 | 6 |
| 3,82 | 47 |
| 3,78 | 92 |
| 3,60 | 80 |
| 3,33 | 37 |
| 3,18 | 31 |
| 2,91 | 8 |
| 2,87 | 81 |
| 2,80 | 5 |
| 2,69 | 9 |
| 2,48 | 7 |

*Beispiel 2*

18,4 g Natriumaluminat (54 Gew.-% $Al_2O_3$, 41 Gew.-% $Na_2O$), 23,5 g Natriumhydroxid und 8,6 g Kaliumhydroxid werden in 130 g Wasser gelöst. In diese Lösung werden unter intensivem Rühren nacheinander zuerst 203 g 40 Gew.-%iges kolloidales Kieselgel, anschliessend 20,4 g Zirkonsulfat $Zr(SO_4)_2$ und zuletzt 4 g Impfkristalle (natürlicher Erionit) eingebracht. Die entstandene Mischung wird homogenisiert und 192 Stunden im geschlossenen Gefäss auf 150° C erhitzt. Das entstandene Produkt wird abfiltriert, mit Wasser gewaschen und bei 120° C getrocknet.

Das Produkt enthält Silicium, Aluminium und Zirkon in folgenden Anteilen, ausgedrückt in Molverhältnissen von Oxiden:

$$SiO_2 : 0,071\ Al_2O_3 : 0,062\ ZrO_2.$$

Die Röntgenbeugungsdaten entsprechen den in Tabelle 1 angegebenen.

**Patentansprüche**

1. Titan-, zirkon und/oder hafniumhaltige Zeolithe, dadurch gekennzeichnet, dass sie
a) ausser Natrium und Kalium die Elemente Silicium, Aluminium sowie mindestens ein Element aus der Gruppe Titan, Zirkon und Hafnium in folgendem Verhältnis enthalten, ausgedrückt in Molverhältnissen von Oxiden:

$$(SiO_2 + MO_2) : (0,02 - 0,30)Al_2O_3,$$

wobei M gleich Titan, Zirkon und/oder Hafnium ist,
und
b) im Röntgenbeugungsdiagramm die folgenden charakteristischen Signale aufweisen:

| Netzebenenabstände d (Å) | relative Intensität $I/I_o$ |
|---|---|
| 11,4 ± 0,3 | stark bis sehr stark |
| 9,2 ± 0,2 | schwach |
| 7,6 ± 0,2 | schwach bis mittel |
| 6,6 ± 0,1 | mittel bis stark |
| 5,7 ± 0,1 | schwach bis mittel |
| 5,35 ± 0,1 | schwach |
| 4,56 ± 0,1 | mittel bis stark |
| 4,32 ± 0,1 | stark |
| 4,16 ± 0,1 | schwach |
| 3,81 ± 0,1 | mittel bis stark |
| 3,75 ± 0,1 | stark bis sehr stark |
| 3,59 ± 0,1 | stark bis sehr stark |
| 3,30 ± 0,1 | mittel |
| 3,15 ± 0,1 | mittel |
| 2,86 ± 0,1 | stark bis sehr stark |
| 2,80 ± 0,1 | schwach bis mittel |

wobei $I_o$ die Intensität des stärksten Signals bedeutet, und bei denen
c) für das Verhältnis Silicium zu Titan, Zirkon und/oder Hafnium gilt:

$$\frac{SiO_2}{SiO_2 + MO_2} = 0,4\text{-}0,99,$$

ausgedrückt in Molverhältnissen der Oxide, wobei M gleich Titan, Zirkon und/oder Hafnium ist.

2. Titan-, zirkon- und/oder hafniumhaltige Zeolithe nach Anspruch 1, dadurch gekennzeichnet, dass für das Verhältnis Silicium zu Titan, Zirkon und/oder Hafnium gilt:

$$\frac{SiO_2}{SiO_2 + MO_2} = 0,7\text{-}0,99,$$

ausgedrückt in Molverhältnissen der Oxide, wobei M gleich Titan, Zirkon und/oder Hafnium ist.

3. Verfahren zur Herstellung von titan-, zirkon- und/oder hafniumhaltigen Zeolithen nach einem der Ansprüche 1 bis 2, dadurch gekennzeichnet, dass man eine Mischung aus Silicium-, Aluminium-, Natrium-, Kaliumverbindungen, Wasser und mindestens einer Verbindung aus der Gruppe der Titan-, Zirkon- und Hafniumverbindungen herstellt, die folgende Zusammensetzung hat, ausgedrückt im Molverhältnissen der Oxide:

$$(SiO_2 + MO_2): (0,02 - 0,30)Al_2O_3: (0,05-0,70)Na_2O: (0,02 - 0,30)K_2O: (5 - 90)H_2O,$$

und wobei für das Verhältnis Silicium zu Titan, Zirkon und/oder Hafnium im Gemisch der Ausgangsverbindungen gilt:

$$\frac{SiO_2}{SiO_2 + MO_2} = 0,4\text{-}0,99,$$

ausgedrückt in Molverhältnissen der Oxide, und wobei M gleich Titan, Zirkon und/oder Hafnium ist, und diese Mischung in einem geschlossenen Gefäss erhitzt.

4. Verfahren nach Anspruch 3, dadurch gekennzeichnet, dass die zu erhitzende Mischung folgende Zusammensetzung hat, ausgedrückt in Molverhältnissen der Oxide:

$$(SiO_2 + MO_2): (0,02-0,18)Al_2O_3: (0,10-0,60)Na_2O: (0,04-0,20)K_2O: (5-40)H_2O,$$

wobei M gleich Titan, Zirkon und/oder Hafnium ist.

5. Verfahren nach einem der Ansprüche 3 oder 4, dadurch gekennzeichnet, dass für das Verhältnis Silicium zu Titan, Zirkon und/oder Hafnium im Gemisch der Ausgangsverbindungen gilt:

$$\frac{SiO_2}{SiO_2 + MO_2} = 0,6\text{-}0,99,$$

ausgedrückt in Molverhältnissen der Oxide, wobei M gleich Titan, Zirkon und/oder Hafnium ist.

6. Verfahren nach einem der Ansprüche 3 bis 5, dadurch gekennzeichnet, dass man der zu erhitzenden Mischung Impfkristalle eines Zeoliths eines ähnliche Strukturtyps zusetzt.

7. Verwendung von titan-, zirkon und/oder hafniumhaltigen Zeolithen nach einem der Ansprüche 1 bis 2 als Katalysatoren bei der Herstellung von $C_2$- bis $C_4$-Olefinen aus Methanol.

## Claims

1. A titanium-, zirconium- and/or hafnium-containing zeolite which
a) contains, in addition to sodium and potassium, the elements silicon and aluminum and at least one element from the group consisting of titanium, zirconium and hafnium in the following ratio, expressed in molar ratios of oxides:

$$(SiO_2 + MO_2): (0.02 - 0.30)Al_2O_3$$

where M is titanium, zirconium and/or hafnium, and
b) has, in the X-ray diffraction diagram, the characteristic signals listed below:

| Lattice plane distances d (Å) | Relative intensity $I/I_0$ |
|---|---|
| 11.4 ± 0.3 | strong to very strong |
| 9.2 ± 0.2 | weak |
| 7.6 ± 0.2 | weak to medium |
| 6.6 ± 0.1 | medium to strong |
| 5.7 ± 0.1 | weak to medium |
| 5.35 ± 0.1 | weak |
| 4.56 ± 0.1 | medium to strong |
| 4.32 ± 0.1 | strong |
| 4.16 ± 0.1 | weak |
| 3.81 ± 0.1 | medium to strong |
| 3.75 ± 0.1 | strong to very strong |
| 3.59 ± 0.1 | strong to very strong |
| 3.30 ± 0.1 | medium |
| 3.15 ± 0.1 | medium |
| 2.86 ± 0.1 | strong to very strong |
| 2.80 ± 0.1 | weak to medium |

where $I_0$ is the intensity of the strongest signal, and in which
c) the ratio of silicon to titanium, zirconium and/or hafnium is:

$$\frac{SiO_2}{SiO_2 + MO_2} = 0.4\text{-}0.99$$

expressed in molar ratios of the oxides, where M is titanium, zirconium and/or hafnium.

2. A titanium-, zirconium- and/or hafnium-containing zeolite as claimed in claim 1, wherein the ration of silicon to titanium, zirconium and/or hafnium is:

$$\frac{SiO_2}{SiO_2 + MO_2} = 0.7\text{-}0.99$$

expressed in molar ratios of the oxides, where M is titanium, zirconium and/or hafnium.

3. A process for preparing a titanium-, zirconium- and/or hafnium-containing zeolite as claimed in any one of claims 1 to 2, which comprises preparing a mixture of silicon, aluminum, sodium and potassium compounds, water and at least one compound from the group consisting of titanium, zirconium and hafnium compounds, which has the following composition as expressed in molar ratios of the oxides:

$$(SiO_2 + MO_2): (0.02 - 0.30)Al_2O_3: (0.05-0.70)Na_2O: (0.02 - 0.30)K_2O: (5 - 90)H_2O,$$

wherein
the ratio of silicon to titanium, zirconium and/or hafnium in the mixture of the starting compounds is:

$$\frac{SiO_2}{SiO_2 + MO_2} = 0.4\text{-}0.99$$

expressed in molar ratios of the oxides, where M is to titanium, zirconium and/or hafnium,
and heating this mixture in a sealed vessel.

4. The process as claimed in claim 3, wherein the mixture to be heated has the following composition as expressed in molar rations of the oxides:

$$(SiO_2 + MO_2) : (0.02 - 0.18)Al_2O_3 : (0.10-0.60)Na_2O : (0.04 - 0.20)K_2O : (5 - 40)H_2O,$$

where M is titanium, zirconium and/or hafnium.

5. The process as claimed in either of claims 3 or 4, wherein the ratio of silicon to titanium, zirconium and/or hafnium in the mixture of the starting compounds is:

$$\frac{SiO_2}{SiO_2 + MO_2} = 0.6\text{-}0.99$$

expressed in molar ratios of the oxides, where M is titanium, zirconium and/or hafnium.

6. The process as claimed in any one of claims 3 to 5, wherein the mixture to be heated has seed crystals of a zeolite of a similar structural type added to it.

7. Use of a titanium-, zirconium- and/or hafnium-containing zeolite as claimed in any one of claims 1 to 2, as a catalyst in the preparation of $C_2$- to $C_4$-olefins from methanol.

## Revendications

1. Zéolites contenant du titane, du zirconium et/ou de l'hafnium, caractérisées en ce que:
a) en plus du sodium et du potassium elles contiennent du silicium, de l'aluminium ainsi qu'au moins un élément pris dans l'ensemble constitué par le titane, le zirconium et l'hafnium, dans le rapport suivant, exprimé en rapports molaires d'oxydes:

$$(SiO_2 + MO_2) : (0,02 - 0,30)Al_2O_3,$$

où M représente le titane, le zirconium et/ou l'hafnium, et en ce que:
b) elles donnent, sur le diagramme de diffraction des rayons X, les signaux caractéristiques qui sont indiqués dans le tableau suivant:

| Distance entre plans réticulaires d (nm) | Intensité relative $I/I_o$ |
|---|---|
| 1,14 ± 0,03 | forte à très forte |
| 0,92 ± 0,02 | faible |
| 0,76 ± 0,02 | faible à moyenne |
| 0,66 ± 0,01 | moyenne à forte |
| 0,57 ± 0,01 | faible à moyenne |
| 0,535 ± 0,01 | faible |
| 0,456 ± 0,01 | moyenne à forte |
| 0,432 ± 0,01 | forte |
| 0,416 ± 0,01 | faible |
| 0,381 ± 0,01 | moyenne à forte |
| 0,375 ± 0,01 | forte à très forte |
| 0,359 ± 0,01 | forte à très forte |

| Distance entre plans réticulaires d (nm) | Intensité relative $I/I_o$ |
|---|---|
| 0,330 ± 0,01 | moyenne |
| 0,315 ± 0,01 | moyenne |
| 0,286 ± 0,01 | forte à très forte |
| 0,280 ± 0,01 | faible à moyenne |

dans lequel $I_o$ désigne l'intensité du signal le plus fort, et en ce que:
c) dans ces zéolites le rapport du silicium au titane, au zirconium et/ou à l'hafnium est tel qu'on ait la relation suivante:

$$\frac{SiO_2}{SiO_2 + MO_2} = 0,4 \text{ à } 0,99,$$

exprimée en rapports molaires des oxydes, le symbole M désignant le titane, le zirconium et/ou l'hafnium.

2. Zéolites contenant du titane, du zirconium et/ou de l'hafnium selon la revendication 1, caractérisées en ce que le rapport du silicium au titane, au zirconium et/ou à l'hafnium est tel qu'on ait la relation suivante:

$$\frac{SiO_2}{SiO_2 + MO_2} = 0,7 \text{ à } 0,99,$$

exprimée en rapports molaires des oxydes, le symbole M désignant le titane, le zirconium et/ou l'hafnium.

3. Procédé de préparation de zéolites contenant du titane, du zirconium et/ou de l'hafnium, selon l'une des revendications 1 et 2, procédé caractérisé en ce qu'on prépare un mélange à partir de composés du silicium, de l'aluminium, du sodium et du potassium, d'eau et d'au moins un composé pris dans l'ensemble des composés du titane, du zirconium et de l'hafnium, mélange qui a la composition suivante, exprimée en rapports molaires des oxydes:

$$(SiO_2 + MO_2) : (0,02 - 0,30)Al_2O_3 : (0,05-0,70)Na_2O : (0,02 - 0,30)K_2O : (5 - 90)H_2O,$$

le rapport du silicium au titane, au zirconium et/ou à l'hafnium dans le mélange des corps de départ étant tel qu'on ait la relation suivante, exprimée en rapports molaires des oxydes:

$$\frac{SiO_2}{SiO_2 + MO_2} = 0,4 \text{ à } 0,99,$$

et le symbole M désignant le titane, le zirconium et/ou l'hafnium, et on chauffe ce mélange dans un récipient fermé.

4. Procédé selon la revendication 3, caractérisé en ce que le mélange à chauffer a la composition suivante, exprimée en rapports molaires des oxydes:

$$(SiO_2 + MO_2) : (0,02 - 0,18)Al_2O_3 : (0,10-0,60)Na_2O : (0,04 - 0,20)K_2O : (5 - 40)H_2O,$$

où M représente le titane, le zirconium et/ou l'hafnium.

5. Procédé selon l'une des revendications 3 et 4, caractérisé en ce que le rapport du silicium au titane, au zirconium et/ou à l'hafnium dans le mélange des corps de départ est tel qu'on ait la relation suivante, exprimée en rapports molaires des oxydes:

$$\frac{SiO_2}{SiO_2 + MO_2} = 0,6 \text{ à } 0,99,$$

où M représente le titane, le zirconium et/ou l'hafnium.

6. Procédé selon l'une quelconque des revendications 3 à 5, caractérisé en ce qu'on ajoute, au mélange à chauffer, des cristaux germes d'une zéolite ayant une structure d'un type analogue.

7. Application de zéolites contenant du titane, du zirconium et/ou de l'hafnium selon l'une des revendications 1 et 2, comme catalyseurs dans la préparation d'oléfines à 2, 3 ou 4 atomes de carbone à partir du méthanol.